# EUROPEAN PATENT APPLICATION

(11) **EP 2 255 765 A1**
(43) Date of publication of application: **01.12.2010**
(21) Application number: 09723462.9
(22) Date of filing: 17.02.2009
(51) Int. Cl.: A61F 13/49, A61F 13/15, A61F 13/53

(54) **APPARATUS AND PROCESS FOR PRODUCING ABSORBENT**

(30) Priority: 17.03.2008 JP 2008068409
(71) Applicant: Uni-Charm Corporation, Ehime 799-0111 (JP)
(72) Inventor: TAKAHASHI, Tatsuo, Kanonji-shi Kagawa 769-1602 (JP); KAMEDA, Noritomo, Kanonji-shi Kagawa 769-1602 (JP)
(74) Representative: Fitchett, Stuart Paul
(86) International application number: PCT/JP2009/052697
(87) International publication number: WO 2009/116344

(57) **Abstract**

Fluid absorbent fibers 2 are accumulated in a form die 21 without being interfered by a polymer charging member 41 for charging a superabsorbent polymer 5.

There is provided an apparatus 10 for manufacturing an absorbent body 1 in which while a form die 21 is moved in one direction along a predetermined moving path, fluid absorbent fibers 2 are distributed toward the form die 21 and accumulated in the form die 21, whereby the absorbent body 1 is formed. A polymer charging member 41 for charging superabsorbent polymer 5 into the form die 21 is disposed to intersect at least a part of a distribution path of the fluid absorbent fibers 2, and a space through which the fluid absorbent fibers 2 are allowed to pass along the distribution path is formed in the polymer charging member 41.

## Description

### Technical Field

The present invention relates to an apparatus and a method for manufacturing an absorbent body of an absorbent article such as a disposable diaper.

### Background Art

As examples of an absorbent article that absorbs fluid, disposable diapers, sanitary napkins, and the like are used. These absorbent articles include an absorbent body that is produced by forming pulp fibers, as an example of fluid absorbent fibers, into a predetermined shape.

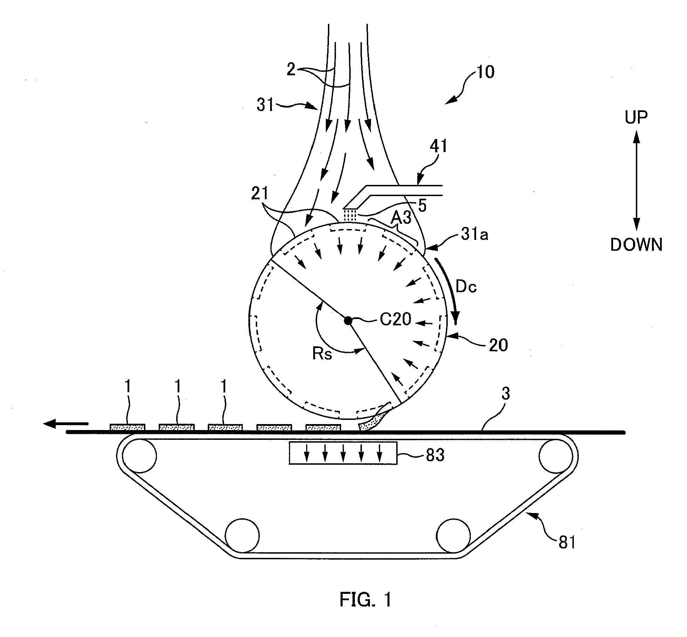

This absorbent body 1 is shaped by a fiber stacking apparatus 10 in a production line (see FIG. 1, for example), and then placed on top of a sheet-like member 3 such as a nonwoven fabric continuously carried on a conveyor 81.

The fiber stacking apparatus 10 includes as the main body a rotating drum 20 that rotates in a clockwise direction, and has recessed form dies 21 formed on the outer circumferential surface of the rotating drum 20. Moreover, a distribution opening 31a of a fiber distributing duct 31 that distributes pulp fibers 2 is provided so as to face the outer circumferential surface. Thus, when a form die 21 passes the position of the distribution opening 31a as the rotating drum 20 rotates, the pulp fibers 2 released in the fiber distributing duct 31 accumulate in the form die 21 and are formed into an absorbent body 1. Thereafter, when the form die 21 reaches a position that faces the conveyor 81, the absorbent body 1 is released from the form die 21 and transferred onto the sheet-like member 3 on the conveyor 81.

Note that a polymer charging member 41 for charging a superabsorbent polymer 5 into the form die 21 is also disposed in the fiber distributing duct 31 (see JP-A-2-107250, for example).

### Disclosure of Invention

### Problem to be Solved by the Invention

In order to charge the superabsorbent polymer 5 uniformly in a width direction (a direction perpendicular to the plane of the paper in FIG. 1), the polymer charging member 41 is formed wide in the width direction. Moreover, since the polymer charging member 41 is set in the fiber distributing duct 31 as described above, the polymer charging member 41 is disposed to intersect at least a part of a distribution path of the pulp fibers 2.

However, with this configuration, a range A3 that is under the polymer charging member 41 is created in the distribution path of the pulp fibers 2, and when the form die 21 passes through the range A3, the accumulation of the pulp fibers 2 is restrained. As a result, there is a risk that the pulp fibers 2 cannot be accumulated as intended.

For example, in order to prevent gel blocking (a phenomenon that may occur in the case where the mixture ratio of the superabsorbent polymer 5 to the pulp fibers 2 is high in an upper layer (the user's skin side) of the absorbent body 1, which is a phenomenon, during absorption of fluid by the absorbent body 1, particles of the superabsorbent polymer 5 in the upper layer swell and join together, and act like a wall so as to inhibit penetration of fluid into a lower layer) of the absorbent body 1, it is preferable that the mixture ratio of the superabsorbent polymer 5 in the upper layer of the absorbent body 1 is low. However, as shown in FIG. 1, in the case where the polymer charging member 41 in the fiber distributing duct 31 extends from a downstream end to the center in a rotating direction Dc (a moving direction of the form die 21) of the rotating drum 20, the position A3 in which pulp fibers 21 of the upper layer of the absorbent body 1 should be accumulated is in the shadow of the polymer charging member 41, and thus the pulp fibers 2 are restrained from reaching the form die 21. As a result, it is difficult to increase the mixture ratio of the pulp fibers 2 in the upper layer.

The present invention was made in light of conventional problems as those described above, and it is an object thereof to provide an apparatus and a method for manufacturing an absorbent body that, even in the case where the polymer charging member for charging the superabsorbent polymer is disposed to intersect at least a part of the distribution path of the fluid absorbent fibers, enable the fluid absorbent fibers to be accumulated in the form die without being interfered by the polymer charging member.

### Means for Solving the Problem

In order to solve the problems as described above, a main aspect of the present invention is an apparatus for manufacturing an absorbent body in which while a form die is moved in one direction along a predetermined moving path, fluid absorbent fibers are distributed toward the form die and accumulated in the form die, whereby the absorbent body is formed, wherein a polymer charging member for charging a superabsorbent polymer into the form die is disposed to intersect at least a part of a distribution path of the fluid absorbent fibers; and a space through which the fluid absorbent fibers are allowed to pass along the distribution path is formed in the polymer charging member.

Another main aspect of the present invention is a method for manufacturing an absorbent body in which while a form die is moved in one direction along a predetermined moving path, fluid absorbent fibers are distributed toward the form die and accumulated in the form die, whereby the absorbent body is formed, wherein a polymer charging member for charging a superabsorbent polymer into the form die is disposed to intersect at least a part of a distribution path of the fluid absorbent fibers; and the fluid absorbent fibers are let through a space formed in the polymer charging member along the distribution path to reach the form die.

Other features of the present invention will become apparent from the description of the present specification with reference to the accompanying drawings.

### Effects of the Invention

According to the present invention, even in a case where the polymer charging member for charging the superabsorbent polymer is disposed to intersect at least a part of the distribution path of fluid absorbent fibers, the fluid absorbent fibers can be accumulated in the form die without being interfered by the polymer charging member.

### Brief Description of Drawings

FIG. 1 is a side view of an apparatus 10 for manufacturing an absorbent body 1 according to the present embodiment of the invention.
FIG. 2 is an enlarged cross-sectional view of a fiber distributing duct 31.
FIG.3A is a side view of a polymer charging member 41, and FIG. 3B is a plan view seen from line B-B in FIG. 3A.
FIG. 4 is an explanatory diagram of another method for shifting discharge openings 41a of the polymer charging member 41 in a circumferential direction Dc.
FIG. 5 is an explanatory diagram of an external shape of pipes 41b of the polymer charging member 41 and are cross-sectional views taken along line V-V in FIG. 3A.
FIGS. 6A to 6D are explanatory diagrams of preferred examples of the external shape.
FIG. 7A is a side view of another embodiment of the polymer charging member 41, and FIG. 7B is a plan view seen from line B-B in FIG. 7A.

### List of Reference Numerals

1 absorbent body, 2 pulp fiber (fluid absorbent fiber), 2a upper layer, 2b lower layer,
3 sheet-like member, 5 superabsorbent polymer, 5a intermediate layer,
10 fiber stacking apparatus (apparatus for manufacturing an absorbent body), 20 rotating drum, 21 form die,
31 fiber distributing duct, 31a distribution opening, 31b opening,
41 polymer charging member, 41a discharge opening, 41b pipe, 41c single pipe,
41d single pipe, 81 belt conveyor, 83 suction box,
S gap (space), H through-hole (space),
A1 first area, A2 second area, A3 third area,
Rs predetermined range, C20 rotation axis, Dc circumferential direction (rotating direction)

### Best Mode for Carrying Out the Invention

At least the following matters will be made clear from the description of the present specification with reference to the accompanying drawings.

An apparatus for manufacturing an absorbent body in which while a form die is moved in one direction along a predetermined moving path, fluid absorbent fibers are distributed toward the form die and accumulated in the form die, whereby the absorbent body is formed,
wherein a polymer charging member for charging a superabsorbent polymer into the form die is disposed to intersect at least a part of a distribution path of the fluid absorbent fibers; and
a space through which the fluid absorbent fibers are allowed to pass along the distribution path is formed in the polymer charging member.

According to such an apparatus for manufacturing an absorbent body, the fluid absorbent fibers reach the form die along the distribution path by passing through the space formed in the polymer charging member. Thus, even when the form die moves to a position under the polymer charging member, the fluid absorbent fibers can be accumulated in the form die without being interfered by the polymer charging member.

In the apparatus for manufacturing an absorbent body, it is desirable that the polymer charging member is a branch pipe in which a discharge opening from which the superabsorbent polymer is discharged is branched into a plurality of pipes; and
the plurality of pipes are aligned in a width direction intersecting both the moving path and the distribution path, and the plurality of pipes are arranged so that adjacent pipes are spaced apart by a gap.

According to such an apparatus for manufacturing an absorbent body, the fluid absorbent fibers reach the position in the shadow of the polymer charging member through the gap between the pipes. In other words, the gap corresponds to the space through which the fluid absorbent fibers are allowed to pass along the distribution path. Thus, even when the form die moves to a position under the polymer charging member, the fluid absorbent fibers can be accumulated in the form die without being interfered by the polymer charging member.

Moreover, since the plurality of pipes are aligned in the width direction, the superabsorbent polymer can also be charged uniformly in the width direction.

In the apparatus for manufacturing an absorbent body, it is desirable that the positions of discharge openings of the plurality of pipes are shifted in a direction along the moving path relative to a discharge opening of a respective adjacent pipe in the width direction.

According to such an apparatus for manufacturing an absorbent body, the positions of adjacent discharge openings in the width direction are shifted from each other in the direction along the moving path, and so the timing at which the superabsorbent polymer is discharged can be staggered between the adjacent discharge openings. As a result, the positions of the superabsorbent polymer that is charged into adjacent positions in the width direction can be shifted in the thickness direction of the absorbent body, and thus gel blocking during absorption of fluid by the absorbent body can thus be effectively prevented.

In the apparatus for manufacturing an absorbent body, it is desirable that the positions of the discharge openings of the plurality of pipes in the direction along the moving path are staggered according to the positions thereof in the width direction.

According to such an apparatus for manufacturing an absorbent body, the positions at which the superabsorbent polymer is charged in the thickness direction of the absorbent body can be evenly arranged without deviation with respect to the width direction.

In the apparatus for manufacturing an absorbent body, it is desirable that an external shape of the plurality of pipes in which a width of an end portion on an upstream side in the distribution path is narrower than a portion on a downstream side from the end portion.

According to such an apparatus for manufacturing an absorbent body, the fluid absorbent fibers can be effectively prevented from collecting at the end portion because the pipes have the above-described external shape, and so the gap through which the fluid absorbent fibers should pass can be reliably secured without clogging.

In the apparatus for manufacturing an absorbent body, it is desirable that the form die is formed on an outer circumferential surface of a rotating drum that rotates in a circumferential direction, and the rotation in the circumferential direction causes the form die to move along a path, as the moving path, in the circumferential direction;
a distribution opening of a fiber distribution duct for distributing the fluid absorbent fibers toward the form die is provided at a predetermined position in the circumferential direction so as to face the outer circumferential surface of the rotating drum; and
the polymer charging member, in the fiber distribution duct, extends from an upstream end toward a downstream side or from a downstream end toward an upstream side in the circumferential direction.

According to such an apparatus for manufacturing an absorbent body, effects of the invention of the present application can be achieved in an effective manner.

A method for manufacturing an absorbent body in which while a form die is moved in one direction along a predetermined moving path, fluid absorbent fibers are distributed toward the form die and accumulated in the form die, whereby the absorbent body is formed, wherein a polymer charging member for charging a superabsorbent polymer into the form die is disposed to intersect at least a part of a distribution path of the fluid absorbent fibers; and
the fluid absorbent fibers are let through a space formed in the polymer charging member along the distribution path to reach the form die.

According to such a method for manufacturing an absorbent body, the fluid absorbent fibers reach the form die along the distribution path by passing through the space formed in the polymer charging member. Thus, even when the form die moves to the position under the polymer charging member, the fluid absorbent fibers can be accumulated in the form die without being interfered by the polymer charging member.

### Embodiment of the Invention

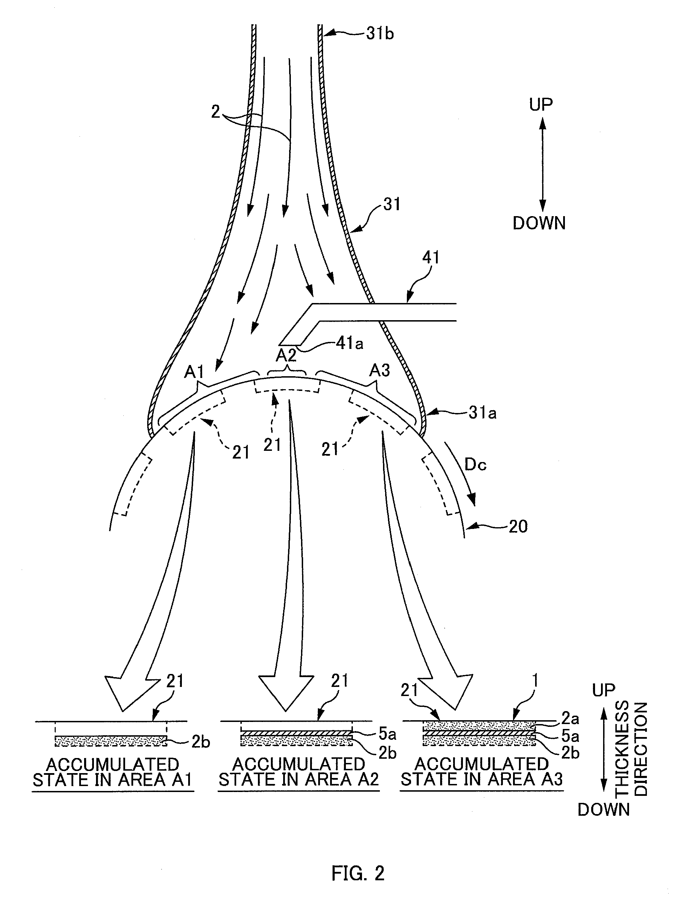

FIGS. 1 and 2 are explanatory diagrams of an apparatus 10 and a method for manufacturing an absorbent body 1 according to an embodiment of the invention. FIG. 1 is a side view of the manufacturing apparatus 10, and FIG. 2 is an enlarged cross-sectional view of a fiber distributing duct 31.

As shown in FIG. 1, the apparatus 10 for manufacturing the absorbent body 1 according to the present embodiment is a so-called fiber stacking apparatus 10, and includes a rotating drum 20 that has recessed form dies 21 formed on an outer circumferential surface thereof and rotates in a circumferential direction Dc, a fiber distributing duct 31 that distributes pulp fibers 2 toward the outer circumferential surface of the rotating drum 20, thereby stacking pulp fibers 2 into the form dies 21 and thus forming absorbent bodies 1, and a belt conveyor 81 that is disposed downstream of the position where the fiber distributing duct 31 is located in the circumferential direction Dc and carries the released absorbent bodies 1 released from the form dies 21.

Note that in the following, the circumferential direction Dc of the rotating drum 20 will be simply referred to as the "circumferential direction Dc", and a width direction of the rotating drum 20 will be simply referred to as the "width direction".

The main body of the rotating drum 20 is, for example, a cylinder body that is driven to rotate in one direction, e.g., the clockwise direction, about a horizontal rotation axis C20. The form dies 21 are formed on the outer circumferential surface thereof at a predetermined pitch in the circumferential direction Dc, and many air suction holes (not shown) are formed at the bottom face of each form die 21. Thus, the pulp fibers 2 in the fiber distributing duct 31 are distributed by riding on an air current produced in the fiber distributing duct 31 by suction of air through the air suction holes and then accumulate in the form dies 21.

Note that the air suction is ceased in a predetermined range Rs at which the form dies 21 face the belt conveyor 81, and in this predetermined range Rs, the absorbent bodies 1 in the form dies 21 are successively released from the form dies 21 by suction of air from a suction box 83 in the carrying conveyor 81 and are transferred onto the belt conveyor 81. Thereafter, the absorbent bodies 1 are carried by the belt conveyor 81.

Incidentally, as shown in FIG. 1, a sheet-like member 3, such as nonwoven fabric, tissue paper, or the like, may be placed on top of the belt conveyor 81 in advance and the absorbent bodies 1 may be transferred onto the sheet-like member 3. In such case, the sheet-like member 3 serves as, for example, a surface sheet (a sheet that is brought into contact with the user's skin) of a disposable diaper or a sanitary napkin.

As shown in FIG. 2, the fiber distributing duct 31 is, for example, a tubular member with a rectangular cross section and is disposed above the rotating drum 20, and a distribution opening 31a at the bottom end thereof covers an upper portion of the outer circumferential surface of the rotating drum 20 over a predetermined range in the circumferential direction Dc. Moreover, the pulp fibers 2 that have been ground by a grinder, which is not shown, or the like are supplied from an opening 31b at the upper end, which is an end on the opposite side of the distribution opening 31a, and thus a path along which the pulp fibers 2 are distributed from above and downward is formed in the fiber distribution duct 31. Thus, when the form dies 21, with the rotation of the rotating drum 20, pass through the position of the distribution opening 31a, the pulp fibers 2 accumulate in the form dies 21 and are formed into absorbent bodies 1.

Incidentally, a polymer charging member 41 for charging a superabsorbent polymer 5 into the form dies 21 is disposed in the fiber distributing duct 31 in close proximity to the outer circumferential surface of the rotating drum 20. A discharge opening 41a of the polymer charging member 41 is positioned approximately at the center of the distribution opening 31a relative to the circumferential direction Dc.

The purpose of employing such an arrangement is, in light of preventing gel blocking during absorption of fluid by the absorbent body 1 and so on, to form an absorbent body 1 into a three-layer structure (see the bottom right accumulated state diagram in FIG. 2) by forming an intermediate layer 5a with a high mixture ratio of superabsorbent polymer 5 in the middle of absorbent body 1 in the thickness direction and forming the upper layer 2a and the lower layer 2b sandwiching the intermediate layer 5a with high mixture ratios of pulp fibers 2.

In other words, first, in a first area A1 in FIG. 2, mainly pulp fibers 2 are accumulated in the form dies 21, and a lower layer 2b mainly composed of the pulp fibers is formed (see bottom left accumulated state diagram in FIG. 2). In a subsequent second area A2, mainly superabsorbent polymer 5 is charged onto the lower layer 2b by the polymer charging member 41, and an intermediate layer 5a with abundant superabsorbent polymer 5 is formed (see bottom center accumulated state diagram in FIG. 2). Then, in a last third area A3, mainly pulp fibers 2 accumulate on the intermediate layer 5a, and the upper layer 2a mainly composed of pulp fibers is formed (see bottom right accumulated state diagram in FIG. 2). Thus, the absorbent body 1 having a three-layered structure is formed.

However, in the case where the discharge opening 41a of the polymer charging member 41 is positioned at the center of the distribution opening 31a in the circumferential direction Dc, either the third area A3 between the center and the downstream end in the circumferential direction Dc of the distribution opening 31a, as shown in FIG. 2, or the first area A1 between the upstream end and the center in the circumferential direction Dc is inevitably located under the polymer charging member 41, and consequently, in either the upper layer 2a or the lower layer 2b, a layer mainly composed of pulp fibers cannot be formed, and the absorbent body 1 would not have the intended three-layer structure.

To address this problem, in the present embodiment, a space along the distribution path of the pulp fibers 2 is formed in the polymer charging member 41, and the pulp fibers 2 are allowed to pass through this space, thereby ensuring pulp fibers 2 to accumulate in the form dies 21 at any position in the third area A3 and the first area A1, which may be located under the polymer charging member 41.

Hereinafter, the polymer charging member 41 according to the present embodiment will be described in detail.

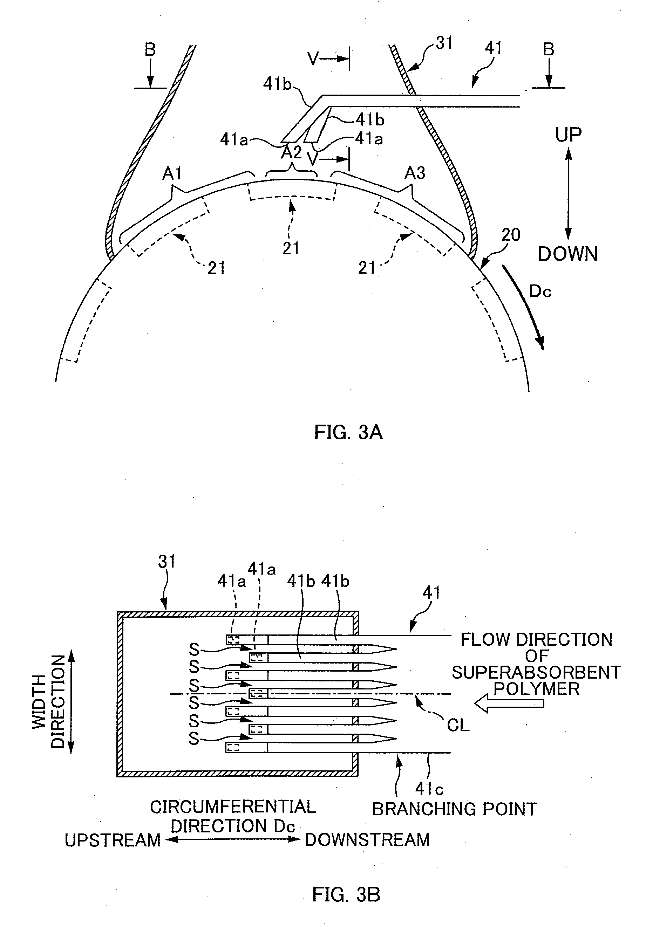

FIG. 3A is a side view of the polymer charging member 41, and FIG. 3B is a plan view seen from line B-B in FIG. 3A.

As can be seen with from FIGS. 3A and 3B, in this example, the polymer charging member 41 is disposed horizontally across the above-described third area A3. Thus, in this example, the polymer charging member 41 may inhibit the pulp fibers 2 from accumulating into the upper layer 2a.

In this regard, in this example, the polymer charging member 41 is configured as a branch pipe in which a portion on the discharge opening 41a side from which the superabsorbent polymer 5 is discharged is branched into a plurality of (seven in the example shown) pipes 41b, and each of the plurality of pipes 41b aligned in the width direction are arranged so that adjacent pipes 41b are spaced apart by a gap S.

Therefore, pulp fibers 2 are made to reach even the form dies 21 moving in the third area A3 in FIG. 3A through the gaps S (corresponding to the aforementioned space). As a result, obstruction of the accumulation of pulp fibers 2 in the upper layer 2a can be effectively minimized. Moreover, since the plurality of pipes 41b are aligned in the width direction, the superabsorbent polymer 5 may be charged uniformly in the width direction.

Here, at least three pipes 41b are disposed in the width direction at a predetermined pitch, and furthermore, the width of the gap S between the pipes 41b is set to 1 mm or more. When three or more pipes 41b are disposed, the distribution of the charged superabsorbent polymer 5 in the width direction can be made further uniform, and the distribution of the accumulated pulp fibers 2 in the width direction can be made uniform. Moreover, when the width dimension of the gap S is set to 1 mm or more, clogging with pulp fibers 2 in the gap S can be minimized. The inner width and the inner height of the discharge opening 41a of each pipe 41b are set within a range of 2 to 50 mm. When the inner width and the inner height are set within such range, the number of pipes 41b can be increased while minimizing clogging with the superabsorbent polymer 5 in each pipe 41b.

Moreover, as shown in FIG. 3B, the positions of the discharge openings 41a of the pipes 41b are shifted in the circumferential direction Dc relative to the discharge opening 41a of a respective adjacent pipe 41b in the width direction. More specifically, the positions of the discharge openings 41a in the circumferential direction Dc are staggered according to the positions thereof in the width direction. The purpose of this is to stagger the timing at which the superabsorbent polymer 5 is charged, between the adjacent discharge openings 41a. In this manner, the positions of the superabsorbent polymer 5 that is charged into adjacent areas in the width direction can be arranged in a vertical zigzag pattern in the thickness direction of the absorbent body 1, and as a result, the joining of particles of the superabsorbent polymer 5, which swell when the absorbent body 1 absorbs fluid, in the width direction can be minimized and gel blocking can thus be effectively prevented.

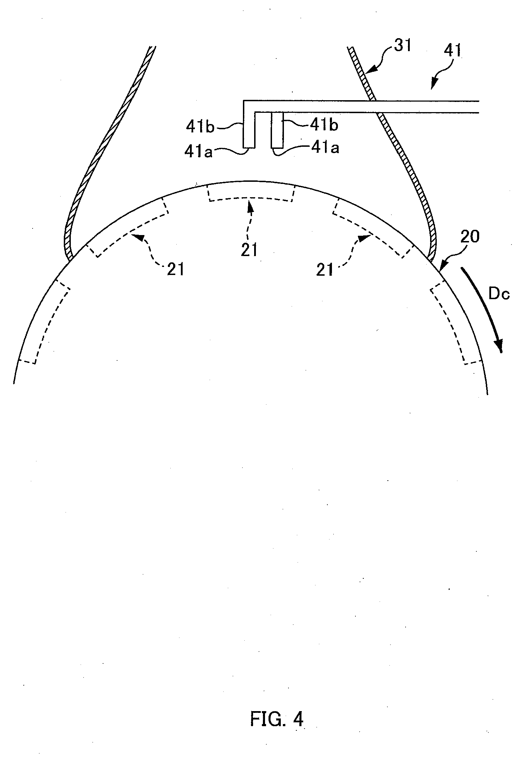

Examples of methods for shifting the positions of the discharge openings 41a in the circumferential direction Dc include a method of setting the angle of inclination from the horizontal line of a portion of each pipe 41b in the vicinity of the discharge opening 41a so that adjacent pipes 41b have mutually different angles of inclination, as shown in FIG. 3A, and a method of directing the portions in the vicinity of the discharge openings 41a of all of the pipes 41b vertically downward and setting the length of each pipe 41b so that adjacent pipes 41b have mutually different lengths, as shown in a side view of FIG. 4.

Note that as shown in FIG. 3B, in the case where the positions of the discharge openings 41a in the circumferential direction Dc are staggered according to the positions thereof in the width direction, it is advantageous to set the number of pipes 41b to an odd number. In this manner, the pipes 41b are arranged in perfect line symmetry with respect to a center line CL in the width direction, and thus, the distribution of accumulated pulp fibers in the width direction can be made further uniform.

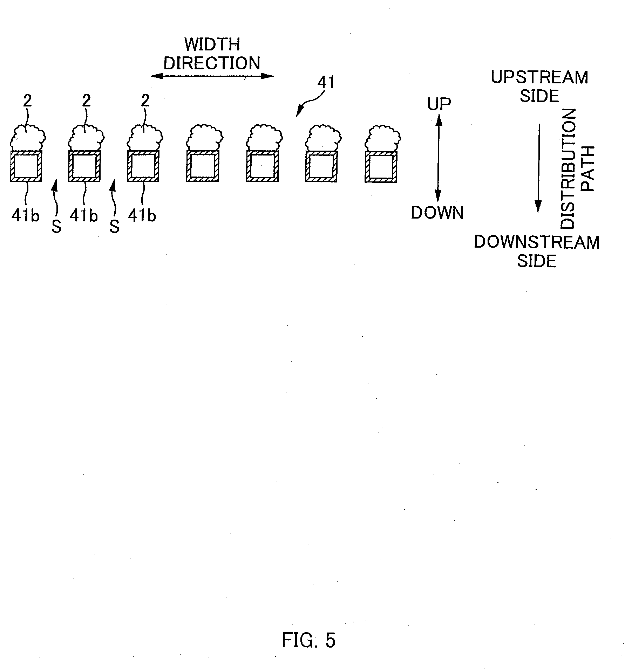

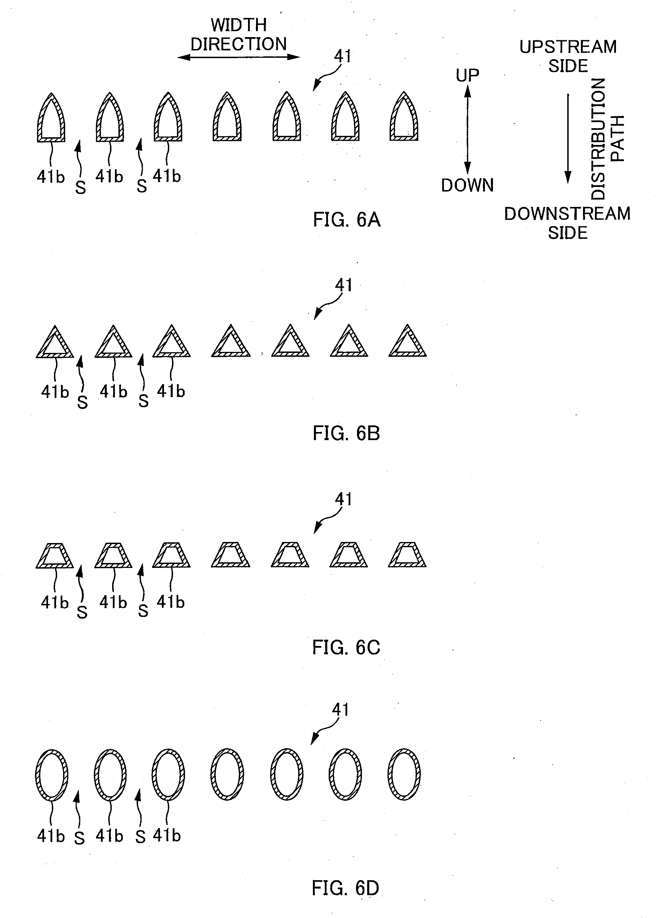

FIG. 5 is an explanatory diagram showing external shapes of the pipes 41b and is a cross-sectional view taken along line V-V in FIG. 3A. In this example, the cross-sectional shape of each pipe 41b when cut in the width direction is rectangular. Preferably, however, it is advantageous that an end portion on the upstream side in the above-described distribution path is narrower than that on the downstream side. Specific examples of such a cross-sectional shape include a streamline shape whose upstream end is tapered as shown in FIG. 6A, a triangular shape whose vertex angle is positioned at the upstream end as shown in FIG. 6B, a trapezoidal shape with the shorter side of the parallel opposite sides of which is positioned upstream as shown in FIG. 6C, and an elliptical shape whose major axis extends along the above-described distribution path as shown in FIG. 6D. With such external shapes, the pulp fibers 2 can be effectively prevented from accumulating on the end portion on the upstream side in the distribution path (see FIG. 5), and induction of clogging of the gap S can be minimized.

Incidentally, the polymer charging member 41 does not necessarily have to be a branch pipe over the entire length thereof, and for example, as shown in FIG. 3B, it is sufficient as long as a portion accommodated in the fiber distributing duct 31 is a branch pipe. Accordingly, a portion located outside the fiber distributing duct 31 may be a single pipe 41c whose pipe path is not partitioned and is continuous as a single communicating hole and the cross-sectional shape of which is wide in the width direction. However, at a branching point, it is advantageous that there is no wall perpendicular to the flow direction of the superabsorbent polymer 5 shown in FIG. 3B. For example, it is desirable that the gap S gradually decreases from the end of the pipes 41b toward the branching point, and finally, all of the pipes 41b are in communication with one another to form a wide single pipe 41c. With such a configuration, accumulation of the superabsorbent polymer 5 on the aforementioned perpendicular wall portion can be avoided, and clogging with the superabsorbent polymer 5 can be effectively prevented.

### Other Embodiments

In the foregoing, an embodiment of the present invention has been described. However, the present invention is not limited to this embodiment, and modifications such as those described below are possible.

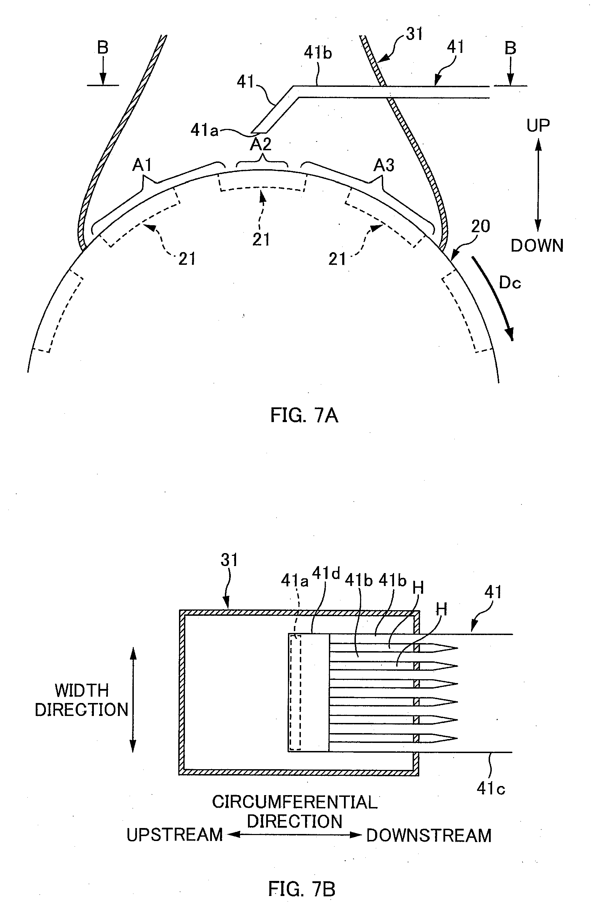

In the foregoing embodiment, as an example, a configuration of a branch pipe as the polymer charging member 41 in which space that allows pulp fibers 2 to pass is formed was described. And as shown in FIG. 3B, each pipe 41b had its own discharge opening 41a. However, the polymer charging member 41 is not limited to such. For example, as shown in FIGS. 7A and 7B, the pipes 41b that have been branched may again be in communication with one another at the position of the discharge openings 41a and form a single pipe 41d that is wide in the width direction. Note that in this case, the polymer charging member 41 has such an appearance that a plurality of through-holes H are formed in a direction along the distribution path of the pulp fibers 2.

In the foregoing embodiment, as an example, a configuration in which the form dies 21 are formed in the outer circumferential surface of the rotating drum 20 where the form dies 21 move along a path in the circumferential direction Dc of the rotating drum 20 was described. However, the configuration is not limited to such, and any configuration can be adopted as long as the form dies 21 move in one direction along a predetermined moving path. For example, it is also possible that the form dies 21 are moved along a predetermined circular path by a chain conveyor or the like, with the fiber distributing duct 31 and the belt conveyor 81 disposed at predetermined positions in the circular path.

In the foregoing embodiment, a fiber distributing duct 31 that is continuous in a vertical direction was disposed above the rotating drum 20, with the distribution opening 31a of the duct covering the outer circumferential surface of the rotating drum 20 from above, thereby forming the distribution path of the pulp fibers 2 that extends in the vertical direction. However, the fiber distributing duct 31 is not limited to such. For example, it is also possible that the fiber distributing duct 31 that is continuous in the horizontal direction or a slanting direction, is disposed lateral to the rotating drum 20 with the distribution opening 31a of the duct laterally covering the outer circumferential surface of the rotating drum 20, thereby forming the distribution path of the pulp fibers 2 that extends in the horizontal direction or the slanting direction.

In the foregoing embodiment, the orientation of the longitudinal direction of the absorbent bodies 1 on the rotating drum 20 was not described in particular. However, this longitudinal direction may be a so-called "machine direction" wherein the longitudinal direction of the absorbent bodies 1 are oriented in the circumferential direction Dc of the rotating drum 20, or a so-called "cross direction" wherein the longitudinal direction of the absorbent bodies 1 are oriented in the width direction of the rotating drum 20.

In the foregoing embodiment, the pulp fibers 2 (pulp that has been ground into fibers) were described as an example of the fluid absorbent fibers. However, cellulose such as cotton, regenerated cellulose such as rayon and fibrillated rayon, semi-synthetic cellulose such as acetate and triacetate, fibrous polymers, and thermoplastic fibers may also be used, or may also be used in combination.

## Claims

1. An apparatus for manufacturing an absorbent body in which while a form die is moved in one direction along a predetermined moving path, fluid absorbent fibers are distributed toward the form die and accumulated in the form die, whereby the absorbent body is formed, the apparatus comprising:
a polymer charging member for charging a superabsorbent polymer into the form die is disposed to intersect at least a part of a distribution path of the fluid absorbent fibers,
wherein a space through which the fluid absorbent fibers are allowed to pass along the distribution path is formed in the polymer charging member.

2. An apparatus for manufacturing an absorbent body according to claim 1,
wherein the polymer charging member is a branch pipe in which a discharge opening from which the superabsorbent polymer is discharged is branched into a plurality of pipes; and
the plurality of pipes are aligned in a width direction intersecting both the moving path and the distribution path, and the plurality of pipes are arranged so that adjacent pipes are spaced apart by a gap.

3. An apparatus for manufacturing an absorbent body according to claim 2,
wherein the positions of discharge openings of the plurality of pipes are shifted in a direction along the moving path relative to a discharge opening of a respective adjacent pipe in the width direction.

4. An apparatus for manufacturing an absorbent body according to claim 3,
wherein the positions of the discharge openings of the plurality of pipes in the direction along the moving path are staggered according to the positions thereof in the width direction.

5. An apparatus for manufacturing an absorbent body according to any one of claims 2 to 4,
wherein an external shape of the plurality of pipes in which a width of an end portion on an upstream side in the distribution path is narrower than a portion on a downstream side from the end portion.

6. An apparatus for manufacturing an absorbent body according to any one of claims 1 to 5,
wherein the form die is formed on an outer circumferential surface of a rotating drum that rotates in a circumferential direction, and the rotation in the circumferential direction causes the form die to move along a path, as the moving path, in the circumferential direction;
a distribution opening of a fiber distribution duct for distributing the fluid absorbent fibers toward the form die is provided at a predetermined position in the circumferential direction so as to face the outer circumferential surface of the rotating drum; and
the polymer charging member, in the fiber distribution duct, extends from an upstream end toward a downstream side or from a downstream end toward an upstream side in the circumferential direction.

7. A method for manufacturing an absorbent body in which while a form die is moved in one direction along a predetermined moving path, fluid absorbent fibers are distributed toward the form die and accumulated in the form die, whereby the absorbent body is formed,
wherein a polymer charging member for charging a superabsorbent polymer into the form die is disposed to intersect at least a part of a distribution path of the fluid absorbent fibers; and
the fluid absorbent fibers are let through a space formed in the polymer charging member along the distribution path to reach the form die.
